# EUROPEAN PATENT APPLICATION

(11) **EP 4 198 027 A1**
(43) Date of publication of application: **21.06.2023**
(21) Application number: 22213255.7
(22) Date of filing: 13.12.2022
(51) Int. Cl.: C07D 239/42, C07D 333/20, C07D 409/12

(54) **SUSTAINABLE PROCESS FOR THE SYNTHESIS OF MOLECULES WITH ANTIHISTAMINE ACTIVITY IN UNCONVENTIONAL BIODEGRADABLE SOLVENTS (DEEP EUTECTIC SOLVENTS)**

(30) Priority: 14.12.2021 IT 202100031322
(71) Applicant: Universita' Degli Studi di Bari, 70121 Bari (IT); Universidad De Oviedo, 33003 Oviedo (ES)
(72) Inventor: QUIVELLI, Andrea Francesca, 70125 Bari (BA) (IT); CAPRIATI, Vito, 70125 Bari (BA) (IT); PERNA, Filippo Maria, 70125 Bari (BA) (IT); VITALE, Paola, 70125 Bari (BA) (IT); ROSSI, Federico Vittorio, 20134 Milano (MI) (IT); GARCÍA - ÁLVAREZ, Joaquín, 33071 Oviedo (ES)
(74) Representative: Trupiano, Federica

(57) **Abstract**

The present invention relates to a sustainable synthetic process for obtaining ethylenediamines molecules with antihistamine activity whose production costs are considerably reduced by employing unconventional biodegradable solvents, such as the Deep Eutectic Solvents, with considerable advantages from a point of view both of the environmental and economic impact.

## Description

### Summary of the Invention

The present invention relates to a sustainable synthetic process for obtaining ethylenediamines molecules with antihistamine activity whose production costs are considerably reduced by employing unconventional biodegradable solvents, such as the Deep Eutectic Solvents, with considerable advantages from a point of view both of the environmental and economic impact.

### Technical background

The massive and extensive use of volatile organic compounds (VOCs), such as the hydrocarbon solvents and other petroleum derivatives, in the pharmaceutical and fine chemical industries, has led to the production of thousands of tons of water per year polluted by organic solvents, as well as has contributed significantly to air pollution. In fact, solvents are often an integral part of the production process of an organic molecule, both because of their ability to solubilize the reagents and because of their influence on the kinetics and thermodynamics of the chemical reactions involved in the production process. Furthermore, solvents are also extensively used for the purification of compounds within extractive, separation, chromatographic and/or crystallization processes.

Today's focus on restricting climate change and environmental impact of the industrial processes has led to the awareness of the need to scale back the VOCs consumption and, at the same time, to move rapidly toward sustainable development linked to careful environmental protection. In particular, since solvents account for about 80-90% of the mass used in a production process, the study, development and subsequent use of so-called *green* solvents as reaction media has become increasingly popular in both industry and academia field. Said "green" solvents share the common characteristic of being low in toxicity, non-flammable, non-explosive, low in cost, widely available and/or readily obtainable from renewable sources.

Among the emerging unconventional solvents that are gradually replacing the VOCs in many fields we may name the so-called "Deep Eutectic Solvents" (DESs).

DESs are eutectic mixtures obtained by combining two or more substances that, when mixed together in appropriate molar ratios, result in the formation of liquid mixtures with a melting point much lower than that of the individual components. Such mixtures generally are constituted by at least one donor and at least one hydrogen bond acceptor, which, because of this characteristic, are strongly associated with each other. This contributes to a significant decrease in the lattice energy of the system, and thus the melting point of the corresponding mixture.

The adjective "deep" is usually used to emphasize large negative deviations that occur for a eutectic mixture with respect to the expected ideal behavior, with a lower than expected eutectic temperature value. For example, by mixing choline chloride (ChCl) (a molecule commonly used as a food additive in chicken feed), which has a melting point (m.p.) of 302 °C, and urea, which has a m.p. of 133 °C, in a 1:2 molar ratio, yields a liquid at room temperature whose m.p. is only 12 °C.

The chemical and physical properties of DESs, which make them particularly attractive for the use as unconventional "green" solvents, include: a nearly zero vapor pressure, high thermal stability, high electrical conductivity, non-flammability, as well as the ability to be recycled. In addition, the compounds that constitute the most common DESs (e.g., amino alcohols, carbohydrates, polyalcohols, organic carboxylic acids, amino acids, vitamins) are often derived from renewable sources. Some specific examples of components commonly used as hydrogen bond acceptors comprise: ChCl, L-alanine, nicotinic acid, glycine. Examples of components that act as hydrogen bond donors include: urea, 1,3-dimethylurea, proline, lactic acid and glycerol. Thanks to these characteristics, the biodegradability of the corresponding eutectic mixtures is extraordinarily high and their toxicity is zero or very low.

In recent years, examples have been reported in the literature about the possible use of these eutectic mixtures in various fields of chemical synthesis. In particular, Saberi D. et al. (Saberi, D. et al. J. Mol. Liq. 2014, 196, 208-210. DOI: 10.1016/j.molliq.2014.03.024), investigated the possibility of carrying out a reductive amination reaction of aldehyde or ketone groups bound to a benzene ring, by using DESs and aromatic amines as solvents. Quivelli A.F. et al. (Quivelli A.F. et al., Front. Chem. 2019, 7, 723. DOI: 10.3389/fchem.2019.00723), have instead developed Ullmann-type coupling reactions catalyzed by copper salts, between heteroaryl halides (Br and I) and aromatic or aliphatic amines, by employing DESs as substitutes for VOCs, which are those normally used. However, both of these works are limited to describing synthetic processes on a laboratory scale, with a final production of the desired compounds in the order of milligrams, while neither the possibility of applying the same methodologies in an industrial setting is investigated, nor is a possible scale-up of the process is carried out. Replication of the synthetic laboratory scheme on a pilot and/or industrial scale requires additional studies and/or modifications in order to maintain high yields, safety, economic benefits and desired characteristics of the final products. So much so that numerous preparatory studies and scale-up calculations, as well as, at times, significant modifications to the process itself are normally carried out, when it becomes necessary to transfer a process from laboratory to industrial scale.

The ethylenediamine antihistamines are a class of compounds whose pharmacological action is based on the antagonism toward the H₁ receptor for histamine, the mediator responsible for the biochemical cascade of the allergic reactions.

Currently, these types of molecules are being industrially synthesized through outdated processes that do not pay much attention to current parameters for environmental and health protection.

US2543544, for example, describes the process of thenyldiamine synthesis from dimethylaminoethylaminopyridine (**A**), 3-thienylbromide and sodium amide, in toluene according to the scheme below, in 30% yield (**Scheme 1**).

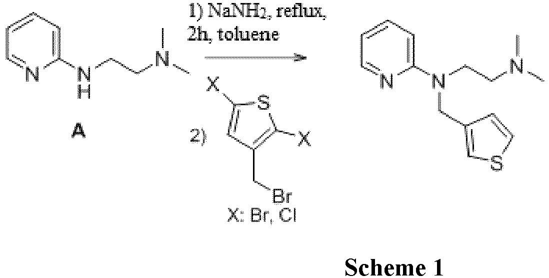

Other examples reported in the literature, for example by Campaigne E. et al. (Campaigne E. et al., J. Am. Chem. Soc. 1954, 76, 2445-2447. DOI: 10.1021/ja01638a041), start from synthetic products, particularly the compound **A** set forth in the previous **Scheme 1** and 5-bromo-thiophene-3-carboxylic acid, and subsequently carry out a reduction with lithium aluminum hydride, thus obtaining the desired compound with an overall yield as low as 8%.

Thus, there is still a great need to provide a synthetic process for this class of molecules that has, compared with the obsolete but still currently used ones, a lower environmental impact, reduced costs, and allows both to obtain the final product with high yields and to produce, in industrially significant quantities, compounds with a high degree of purity.

### Objects of the invention

Object of the present invention is to provide a novel method of synthesizing molecules with antihistamine activity and, in particular, ethylenediamine antihistamines, which is (a) suitable for large-scale industrial production, (b) environmentally friendly and low-cost, and (c) enables the molecules with antihistamine activity to be obtained in high yields.

Further object of the present invention is to provide molecules with antihistamine activity that are obtained through an industrially applicable synthetic process with low environmental impact and cost-effectiveness.

These and other objects are achieved by what is the subject matter of the present invention, which relates to a novel synthetic process for molecules with antihistamine activity.

### Description of the invention

Subject matter of the present invention is an environmentally sustainable method of synthesizing molecules with antihistamine activity, in particular ethylenediamine antihistamines of Formula I, which uses solvents of the DESs class, as previously described, in place of normally used VOCs. 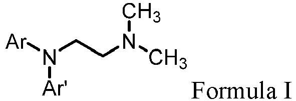 where Ar and Ar' have the meanings given in **Table 1**, respectively.

**Table 1: Meanings of Ar and Ar' in Formula I and corresponding name of the resulting molecule with antihistamine activity.**

| Ar | Ar' | name |
|---|---|---|
| 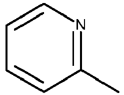 | 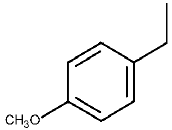 | Mepyramine |
| 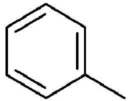 | 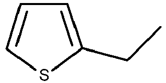 | Metaphenylene |
| 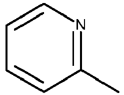 | 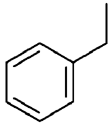 | Tripelennamine |
| 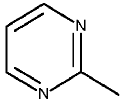 | 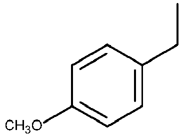 | Thonzylamine |
| 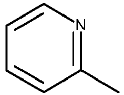 | 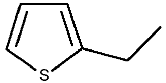 | Metapyrylene |
| 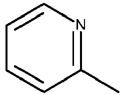 | 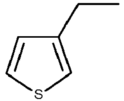 | Thenyldiamine |

The synthesis methods currently available and used in industry settings for the production of Formula I compounds are characterized, as previously mentioned, by poor economics of the process as well as a total lack of consideration of any principles that may be part of the concept of "green chemistry" that is desired and pursued today. Furthermore, they are synthetic routes that have the inherent risk linked to the use of toxic and flammable reagents and solvents, as well as are characterized by an Environmental Factor (E-factor) that is not very acceptable today.

The synthesis method subject matter of the present invention solves these and other problems currently existing, making the production of this class of compounds environmentally sustainable thanks to the use of DESs and the possibility to recycle them within the same process, while increasing the overall economics of the reaction.

According to an aspect of the present invention, the synthesis of the ethylenediamine derivatives of Formula I occurs in two distinct steps; said steps both use DESs as solvents.

In particular, the DESs used as solvents in the desired reactions, which may be prepared extemporaneously prior to starting or in advance to the synthesis process itself and may be stored for long periods of time, are obtained by mixing in precise molar ratios two or more components that will constitute the DES of choice for the reaction to be carried out, by heating to a temperature of 60-80 °C.

According to a preferred aspect of the present invention, the DESs of choice to be used in place of the conventional solvents are constituted by one of the following compounds defined as hydrogen bond acceptors: ChCl, L-alanine, nicotinic acid and glycine; and one of the following compounds, defined as hydrogen bond donors: urea, 1,3-dimethylurea, proline, lactic acid and glycerol. ChCl/urea and ChCl/glycerol pairs are particularly preferred.

The first step in the synthesis according to the present invention, and shown in **Scheme 2** below, is a reductive amination that takes place in DESs from an aromatic aldehyde and *N,N*-dimethylethan-1,2-diamine, in the presence of a reducing agent, preferably sodium borohydride (NaBH₄), at room temperature, resulting in the corresponding amine, where Ar may have any of the meanings previously set forth in **Table 1.**

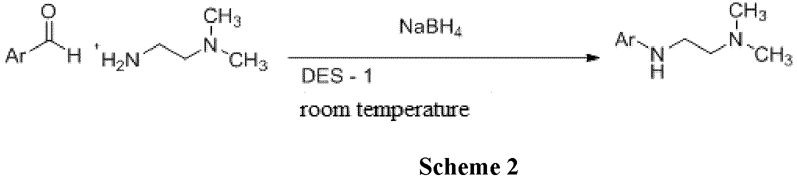

According to a preferred aspect of the invention, the first step described in **Scheme** 2 uses as DES both the ChCl/urea mixture and the ChCl/glycerol mixture, in 1:2 molar ratios to each other; preferably, it uses ChCl/glycerol.

According to a further aspect of the present invention, the reaction of this first step lasts between 2 and 4 hours.

The amine obtained from the first step of the synthesis process of the present invention may be isolated by using the techniques known to the skilled in the art. According to a particular aspect of the present invention, it is preferably isolated through an acid-base extraction in water/organic solvent.

According to the present invention, the yield obtained from this first synthetic step is very high, higher than 95% and up to ≥ 98%.

The second step of the synthesis, according to the present invention, is shown in **Scheme 3** and is constituted by an Ullmann-type coupling reaction, catalyzed by copper salts, in a suitably selected DES, which is made to occur between the amine produced in the first step of the reaction according to **Scheme 2** above and an aryl bromide.

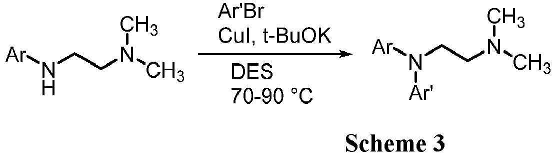

According to a preferred aspect of the invention, the second step described in **Scheme 3** uses as DES the ChCl/urea mixture or the ChCl/glycerol mixture; preferably, it uses ChCl/glycerol.

According to a preferred aspect of the invention, the catalyst used for said coupling reaction is copper iodide. In fact, this compound is an inexpensive source of copper that is nevertheless used in catalytic quantities. The reaction constituting the second step of the synthesis, according to the present invention, involves the addition of a base, preferably potassium *tert*-butylate (t-BuOK) added in amounts between 2 and 4 equivalents; preferably, 3 equivalents.

The step described in **Scheme 3** is carried out between 70 and 90 °C; preferably, at 80 °C. The reaction is carried out for a total of 20-30 hours.

According to the present invention, the yield of the second reaction step, according to **Scheme 3,** leads to a yield of the final product of 40-45%.

Furthermore, the purification of the synthetic product of interest (Formula I) does not involve the use of additional organic solvents. The simple addition of water at the end of the reaction leads to the separation of the desired compound (as a dark oily phase) from the aqueous phase by simple centrifugation.

According to a particularly preferred aspect, the unreacted synthesis substrate, i.e., the amine resulting from the reaction of the first reaction step (**Scheme 2**), may be easily recovered and reused. This aspect also contributes to making the synthesis process of the present invention particularly economical and characterized by a low environmental impact.

Preferably, the recovery of the unreacted amine, in the second step of synthesis according to the present invention, takes place by its conversion to hydrochloride and subsequent precipitation as a white solid by using hydrochloric acid in isopropanol.

According to a preferred aspect, the process object by the present invention may be easily implemented not only to produce small quantities of molecules with antihistamine activity on a laboratory scale but is also suitable for industrial-scale production, as will be shown in the Experimental section below.

The industrial-scale applicability of the present invention is particularly important from the point of view of the advantages the process brings over those currently belonging to the state of the art. In fact, if one considers the purely economic aspect, a comparison of the production costs of the antihistamine molecules of interest obtained by the synthesis process according to the present invention, against their current selling price, may be made. For example, thenyldiamine has a market cost at the time of filing the present application of about 300 €/50 mg. In the case of using the synthesis route according to the process object of the present invention, the costs of the final product thenyldiamine, calculated as the sum of the costs of the individual reagents used, is about 0.365 €/50 mg, (for what concerns raw materials only), as per Example 1 set forth in the Experimental section below.

When compared with the current state of the art, the process object of the present invention also has other particularly favorable aspects, in addition to the previously described advantages and the possibility of reducing costs by achieving higher yields, fewer steps and a more efficient purification stage. Particularly noteworthy is the fact that non-toxic solvents are used in the process of the invention, which therefore do not need to be stored according to special safety protocols, and may be handled easily and safely by operators, also having fewer disposal problems. The same applies to the reducing agent of choice used for the synthesis according to the present invention, namely NaBH₄. The latter is a mild reducing agent that allows the synthesis of antihistamine derivatives to be completed and is therefore particularly suitable to take the place of a more vigorous reducing agent such as LiAlH₄, which is the one stated in the literature. The latter not only shows difficulties in its use but its use poses dangers related to operator safety.

The possibility of recovering the primary amine, as previously described (i.e., the starting reagent of the second step of the synthesis according to the present invention), is a further significant advantage of the method of the invention, not only for economic reasons, but also in relation to effects related to greater environmental friendliness and lower consumption of raw materials.

Furthermore, the procedure for purifying the desired product at the end of the reaction, still according to the process object of the present invention, is very simple as it does not require the use of additional organic solvents. Once the second step of the synthesis is completed, according to the present invention, after the addition of water, the product of interest may be separated by simple centrifugation, thanks to which the antihistamine derivative is separated as an oily phase.

Finally, it is important to remember that the components that constitute the DESs and in particular ChCl, urea and glycerol, are to be considered *"commodities", i.e.*, chemical substances produced in large quantities, in dedicated plants and by continuous processes. For this reason, they are generally low in cost and there is no need to design their synthesis because they are compounds that are always readily available on the market.

The present invention thus solves technical problems related to the synthesis of molecules with antihistamine activities, to date, still present and felt in industry, in particular, with reference to production costs and environmental impact.

The following Experimental section aims to exemplify, in a non-limiting way, the present invention by illustrating some of its specific aspects.

### Experimental section

### Example 1 - Synthesis of thenyldiamine according to the present invention

The synthesis of an antihistamine compound, in particular thenyldiamine (compound **4**), is implemented by using the method described in the present invention according to the following steps:

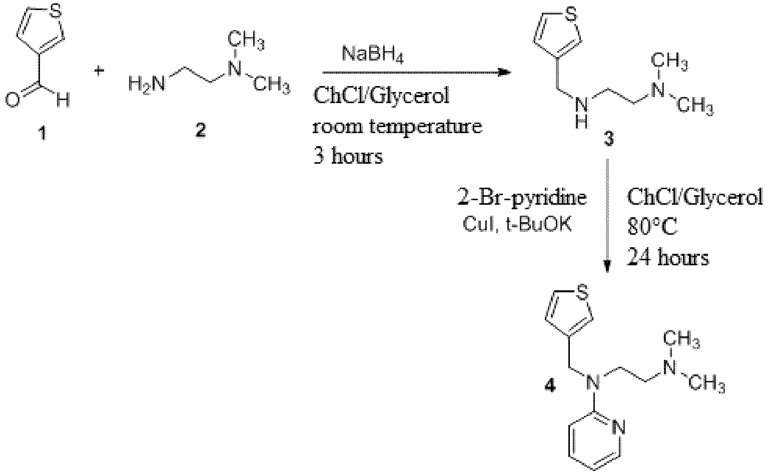

56 mg of compound **1** is reacted with 44 mg of compound **2** in 1 g of a 1:2 mol/mol mixture of ChCl/glycerol, previously prepared by mixing 0.57 g of glycerol and 0.43 g of ChCl and heating to 60 °C, adding 18.9 mg of NaBH₄ as the reducing agent. The reaction continues for 3 h at room temperature with the formation of the secondary amine (compound **3**), isolated by extraction with water/cyclopentyl methyl ether (CPME), with a yield of 98%.

Compound **3** is then suspended in 1 g of a 1:2 mol/mol mixture of ChCl/glycerol, prepared by mixing 0.57 g of glycerol and 0.43 g of ChCl and heating to 60 °C, adding 79 mg of 2-bromopyridine, 9.5 mg of CuI and 168 mg of *t*-BuOK. The reaction is continued for 24 h at 80 °C, obtaining 51.6 mg of the desired compound **4,** which is isolated by addition of water, centrifugation and separation of the dark oily phase that constitutes compound **4.**

The cost of reagents and solvents used to produce 51.6 mg of thenyldiamine is set forth in **Table 2** below.

**Table 2: Production costs of thenyldiamine according to the process object of the present invention.**

| reagent/solvent | quantity | cost (€)^{a} |
|---|---|---|
| ChCl/glycerol | 1 g | 0.030 |
| **1** | 56 mg | 0.051 |
| **2** | 44 mg | 0.015 |
| NaBH₄ | 18.9 mg | 0.004 |
| CPME | 3 mL | 0.15 |
| ChCl/glycerol | 1 g | 0.030 |
| 2-bromopyridine | 79 mg | 0.049 |
| CuI | 9.5 mg | 0.002 |
| *t*-BuOK | 168 mg | 0.034 |
| total for 51.6 mg of compound **4** | | 0.365 |

| | | |
|---|---|---|
| ^{a} ChCl/glycerol (1:2 mol mol⁻¹); ChCl: 29 €/500 g (TCI); glycerol: 12,20 €/1 L (density: 1,26 g/mL) (Alfaesar); NaBH₄: 118 €/500 g (TCI); CPME: 30,5 €/500 g (density: 0,86 g/mL) (Fluorochem); CuI: 122 €/500 g (TCI); *t*-BuOK: 102 € /500 g (TCI). | | |

### Example 2 - Scale-up of the synthesis of thenyldiamine according to the present invention

The synthetic process previously illustrated in Example 1 has been reproduced at a scale up to 1,000 times larger in the research and development laboratory of a company producing pharmaceutical active ingredients.

In particular, the scale-up process was performed in the quantities and proportions set forth in **Table 3** below.

**Table 3: Scale-up of the synthesis of thenyldiamine up to a 1:1000 scale.**

| scale | compound **1** g (mmol) | compound **2** | ChCl/glycerol g | compound **3** g (yield%) | ChCl/glycerol g | thenyldiamine g (yield%) |
|---|---|---|---|---|---|---|
| x1 | 0.05 (0.45) | 1 equivalent | 1 | 0.081 (98) | 1 | 0.046 (40) |
| x20 | 1 (9) | 1 equivalent | 10 | 1.62 (98) | 10 | 0.92 (40) |
| x200 | 10 (90) | 1 equivalent | 100 | 16.2 (98) | 100 | 9.2 (40) |
| x1,000 | 50 (450) | 1 equivalent | 500 | 81 (98) | 500 | 45.9 (40) |

Starting with 50 mg of the starting aldehyde (compound **1**) in 1 g of DES (ChCl/glycerol) as described in Example 1, a gradual scale-up was carried out until 50 g of compound **1** was used in 500 g of DES, resulting in the production of 81 g of intermediate **3**, with an unchanged yield of 98%, and 45.9 g of thenyldiamine (unchanged yield of 40%).

In conclusion, therefore, it has been demonstrated that it is possible to reproduce the synthesis process object of the invention even on a scale 1,000 times larger than the laboratory scale by which it was developed, without in any way affecting either the yield or the purity of the final compound.

### Example 3 - Synthesis of thonzylamine according to the present invention

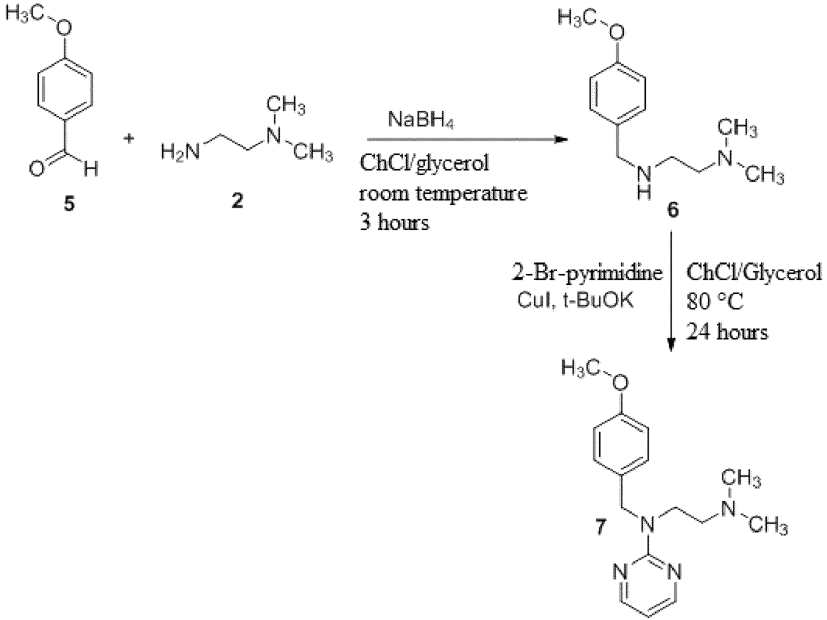

68 mg of compound **5** is reacted with 44 mg of compound **2** in 1 g of a 1:2 mol/mol mixture of ChCl/glycerol, previously prepared by mixing 0.57 g of glycerol and 0.43 g of ChCl and heating to 60 °C, adding 18.9 mg of NaBH₄ as the reducing agent. The reaction continues for 3 h at room temperature with the formation of the secondary amine (compound **6**) isolated by extraction with water/ethyl acetate, with a yield of 98%.

Compound 6 is then suspended in 1 g of a 1:2 mol/mol mixture of ChCl/glycerol, prepared by mixing 0.57 g of glycerol and 0.43 g of ChCl and heating to 60 °C, adding 79 mg of 2-bromopyrimidine, 9.5 mg of CuI and 168 mg of *t*-BuOK. The reaction is continued for 24 h at 80 °C, obtaining 56 mg of the desired compound 7, which is isolated by addition of water, centrifugation and separation of the dark oily phase that constitutes compound 7.

### Example 4 - Synthesis of metaphenylene according to the present invention

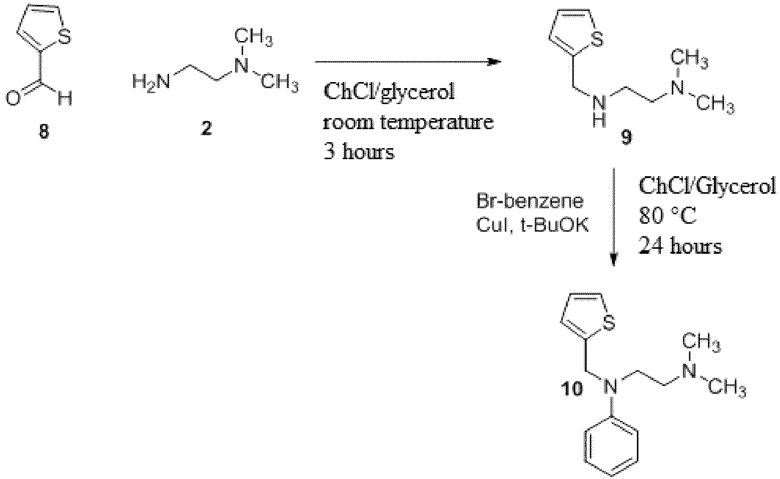

56 mg of compound **8** is reacted with 44 mg of compound **2** in 1 g of a 1:2 mol/mol mixture of ChCl/glycerol, previously prepared by mixing 0.57 g of glycerol and 0.43 g of ChCl and heating to 60 °C, adding 18.9 mg of NaBH₄ as the reducing agent. The reaction continues for 3 h at room temperature with the formation of the secondary amine (compound **9**) isolated by extraction with water/CPME, with a yield of 98%.

Compound **9** is then suspended in 1 g of a 1:2 mol/mol mixture of ChCl/glycerol, prepared by mixing 0.57 g of glycerol and 0.43 g of ChCl and heating to 60 °C, adding 78 mg of bromobenzene, 9.5 mg of CuI and 168 mg of *t*-BuOK. The reaction is continued for 24 h at 80 °C, obtaining 51 mg of the desired compound 7, which is isolated by addition of water, centrifugation and separation of the dark oily phase that constitutes compound **10.**

## Claims

1. A process for synthesizing ethylenediamine compounds having antihistamine activity, **characterized in that** it uses DESs as solvents.

2. The process according to claim 1, **characterized in that** said DESs are constituted by the mixture of a compound selected from: choline chloride (ChCl), L-alanine, nicotinic acid or glycine, and a compound selected from: urea, 1,3-dimethylurea, proline, lactic acid or glycerol.

3. The process according to claim 2, **characterized in that** said DESs are constituted by the ChCl/glycerol pair.

4. The process according to claim 1, **characterized in that** said ethylenediamine compounds are of Formula I 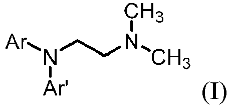 where Ar and Ar' are:
| Ar | Ar' | name |
|---|---|---|
| 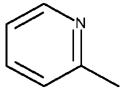 | 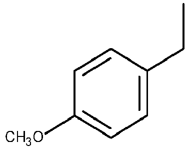 | Mepyramine |
| 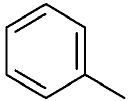 | 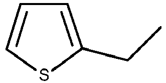 | Metaphenylene |
| 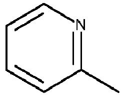 | 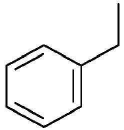 | Tripelennamine |
| 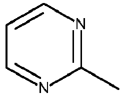 | 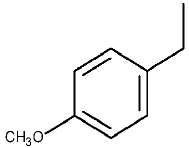 | Thonzylamine |
| 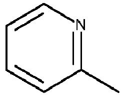 | 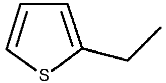 | Metapyrylene |
| 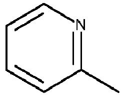 | 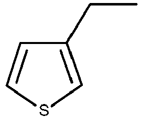 | Thenyldiamine |

5. The process according to claim 1, **characterized in that** it comprises a first step of reductive amination in DESs of an aromatic aldehyde with *N,N-*dimethylethan-1,2-diamine in the presence of a reducing agent.

6. The process according to claim 5, **characterized in that** said reducing agent is sodium borohydride (NaBH₄).

7. The process according to claim 5, **characterized in that** it comprises a second Ullman-type coupling step with catalyst based on a copper salt in DES of an amine with an aryl bromide.

8. The process according to claim 7, **characterized in that** said copper catalyst is the copper iodide.

9. The process according to claim 7, **characterized in that** it comprises adding a base, preferably potassium *tert*-butylate (*t*-BuOK).

10. Use of DESs as solvents for the preparation of ethylenediamine compounds having antihistamine activity.
